# EUROPEAN PATENT SPECIFICATION

(11) **EP 0 572 932 B1**
(45) Date of publication and mention of the grant of the patent: **13.09.2000**
(21) Application number: 93108636.7
(22) Date of filing: 28.05.1993
(51) Int. Cl.: A61K 9/00, A61M 31/00

(54) **Method and implant device for long term delivery of drugs**
Verfahren und Implantatvorrichtung für Langzeitwirkstoffabgabe
Procédé et dispositif implantable pour la délivrance à long terme de médicaments

(30) Priority: 02.06.1992 US 892204
(43) Date of publication of application: 08.12.1993
(73) Proprietor: C.R. BARD, INC., Murray Hill New Jersey 07974 (US)
(72) Inventor: Lee, Clarence C., Lilburn, Georgia 30247 (US)
(74) Representative: Sternagel, Hans-Günther, Dr.

(56) References cited:
- EP-A- 0 293 632
- EP-A- 0 303 306
- EP-A- 0 417 572
- WO-A-92/02211

## Description

The present invention relates to a device for delivering therapeutic agents to a tissue or organ. More particularly, the present invention relates to a device for the long-term delivery of a drug or a plurality of drugs directly to a target smooth muscle tissue or organ.

### Background of the Invention

Many organs and glands in the body contain muscles that differ from skeletal muscle in not being under voluntary control and in not having a striated or banded appearance under the light microscope. These smooth muscles lack striations because their contractile filaments are not organized in regular arrays of aligned sarcomeres. Smooth muscles are usually specialized for long, slow contractions. Rather than taking instructions from motor neurons with cell bodies in the central nervous system, they are controlled by nerve fibers of the autonomic nervous system and by circulating hormones.

Smooth muscles perform a wide variety of functions. They control the diameter of arterioles and thus help regulate pressure. They form muscular sphincters at branches of the vascular tree and thus determine the distribution of blood to different capillary beds. Gastrointestinal sphincters control passage of intestinal contents from one section of the gut to the next. Smooth muscles regulate the size and internal pressure in hollow organs such as the urinary bladder and the uterus. Smooth muscles vary greatly in electrical activity, degree of automaticity, innervation, response to the circulating hormones and drugs, and extent of cell-to-cell coupling. (For a review of smooth muscles, see Stephens, N.L., *Smooth Muscle Contraction,* New York, M. Dekker, (1984)).

In unitary smooth muscle, contraction is synchronized so that the muscle acts as a unit, all fibers either contracting or resting. Unitary muscles are spontaneously active, display electrical pacemaker activity, and respond to stretch with increased activity. The innervation density is low, and the cells are tightly coupled electrically through gap junctions so that activity, once initiated, spreads promptly from cell to cell. The multicellular muscle acts as a single unit. Unitary smooth muscle thus resembles cardiac muscle more closely than striated muscle. Examples are the gut and uterus, organs that generate and propagate their own slow, rhythmic movements.

Time release delivery of drugs to a smooth muscle tissue or organ is desired where there is a requirement for long-term administration of the drug to the tissue or organ. In addition, it is often advantageous to be able to deliver a drug directly to the site which needs to be treated and thereby avoid systemic administration of the drug. This is especially true where the drug may be harmful to certain tissues or organs if administered systemically. Localized delivery would allow one to deliver high concentrations of the desired drug to a specific region of the body and yet not harm other tissues and organs.

One of the uses for the "time-release" delivery of drugs to a specific tissue or organ is for internal organs that can be classified as mechanically dynamic organs. Examples of mechanically dynamic organs include, but are not limited to, the stomach, intestines, heart, bladder, ureter, urethra, various sphincter muscles, and the esophagus. The functions of these organs are maintained by their normal and timely mechanical contractions. Should the mechanical properties be lost, the intended functions of these organs are partially or completely lost.

The mechanical contractions are dependent on the states of the constituent smooth muscle and the nerve systems involved. Factors which would affect the contractions of these organs include anxiety, biochemical or hormonal imbalance, smooth muscle disease or dysfunction, and lesions of central or peripheral nerves. A specific example can be seen when stress can ultimately effect one's stomach contractions thereby causing ulcers in some individuals. Uterine cramps in women can be caused by anxiety, biochemical and/or hormonal imbalances. Peripheral neural lesions can cause urge incontinence.

Currently available therapies designed to externally regulate the contraction of internal organs are limited to electro-stimulation of specific diseased organs and/or systemic administration of musculotropic agents and/or neurochemicals. The implantation of a permanent electro-stimulator exposes patients to potential risks associated with major surgery and general anesthesia. The malfunctioning or infection of the electro-stimulation implant can further complicate or reverse the prognosis of these patients. It is well known that systemic administration of neurochemicals and/or musculotropic agents would adversely affect other non-diseased organs and tissues. The possible side effects can include dry mouth, blurred vision, mydriasis, tachycardia, drowsiness and constipation. In general, patients with glaucoma or a heart condition are excluded from systemic administration of these types of agents. Thus, the localized controlled administration of neurochemicals and/or musculotropic agents would optimize their effects on the targeted organs without significantly affecting other organs or nerve systems.

Several patents describe short-term delivery compositions and devices. U S -A- 4,913,903 discloses a biodegradable polymer which can be admixed with a therapeutic agent and implanted into the body allowing short-term release of a therapeutic agent. The polymer is based upon two polyorthoesters and forms a solid polymer. The polymer disclosed in the '903 patent is designed to release chemotherapeutic agents over a relatively short period of time such as the convalescent period after surgery. The biodegradable polymer disclosed in the '903 patent would not be useful in the situation where long-term administration, i.e., one to two years, is necessary.

U S -A- No. 4,871,542 discloses a method and apparatus for delivering therapeutic agents to the interior of the bladder and the urinary tract. The device disclosed in the '542 patent is a porous, minicellular polymeric container which acts as a reservoir for a therapeutic agent. The size of the minicellular pores regulates the rate of diffusion of the therapeutic agent. The device described in the '542 patent would not be suitable for implantation into a tissue for the long-term administration of a therapeutic agent.

U S -A- 4,775,659 discloses an injectable, time-release formulation comprising a therapeutic agent, an oil and a suitable glyceride release modifying agent. The composition is injected intramuscularly, and the therapeutic agent is released over a period of days. The formulation described in the '659 patent would not be appropriate for the long-term delivery of a therapeutic agent to a tissue or organ.

U S -A- 4,725,442 discloses injectable microdroplets containing water-insoluble drugs. The preferred use of the microdroplets is the localized, time-release delivery of anesthetics. Again, the release of the water-insoluble drug from the microdroplets is over a period of days. The microdroplets disclosed in the '442 patent would not be suitable for release of a biologically active agent over a period of months to years.

From EP-A-303 306 an implant of polymeric material is known which can release a contraceptive agent for a relatively long time. The implant comprises a core material of ethylene/vinyl acetate copolymer as matrix for the contraceptive agent and a membrane consisting of the same copolymer.

EP-A-293 632 refers to a delivery system for the controlled administration of LHRH analog comprising a silicon elastomer matrix which is implantable.

In EP-A-417 572 an implantable polymeric device is disclosed which releases biologically active materials. The matrix material of the device is ethylene/vinyl acetate copolymers or polymers selected from acyl substituted cellulose acetates and alkyl derivatives thereof; partially and completely hydrolyzed alkylene/vinyl acetate copolymers, unplasticized polyvinyl chloride, crosslinked homo- and copolymers of polyvinyl acetate, crosslinked polyesters of acrylic and methacrylate; polyvinyl alkyl ethers; polyvinyl fluoride, silicone; polycarbonate, polyurethane; polyamide, polysulphones; styrene acrylonitrile copolymers, crosslinked poly(ethylene oxide); poly(alkylenes); poly(vinyl imidazole); poly(esters); poly(ethylene terephthalate); and chlorosulphonated polyolefins.

It is the object of the invention to provide an injectable device which can be implanted directly into the target tissue and which is capable of releasing a biologically active agent with preferably zero order kinetics over a period of months or years. Such an injectable device would be particularly effective in treating patients with bladder instability etiology.

This object is attained by an implant device for the long term delivery of a therapeutic agent to a tissue or organ comprising (i) a container having a wall for implantation within the tissue or organ and (ii) a therapeutic agent immobilized in a matrix contained within the container such that the therapeutic agent is released into the tissue or organ at a rate of at least approximately 0.1 µg/day over a period of at least approximately three months; and which is characterized in that the matrix is biodegradable and selected from liposomes, polyactides, polyanhydrides, polyglycolide, poly(lactide co-glycolide), polypeptides, hyaluronic acid, collagen, chondroitin sulfate, carboxylic acids, fatty acids, phospholipids, polysaccharides, nucleic acids, phenylalanine, tyrosine, isoleucine, polynucleotides, polyethylene glycol, polyvinylpropylene, polyvinylpyrrolidone and beeswax. The biodegradable matrix is admixed with a therapeutic agent. The container can be implanted into a tissue that would benefit from the long-term administration of the therapeutic agent.

More particularly, the present invention is an implant device for the long term delivery of a therapeutic agent to a tissue or organ, comprising a container for implantation within the tissue or organ, a therapeutic agent contained within the container; and means associated with at least one of the container and the therapeutic agent for controlling the rate of release of the therapeutic agent from the container into the tissue or organ such that the therapeutic agent is released at a rate of between approximately 0.1 µg to approximately 12 mg/day over a period of at least three months.

In a preferred embodiment, the container is a cylinder with at least one opening to the tissue in which the cylinder is implanted. The cylinder can be manufactured from silicone, stainless steel, titanium or other types of medical/surgical grade plastics or metals. Plastics that can be used in constructing the present invention include, but are not limited to, polyethylene, polypropylene and parylene. The cylinder walls are impermeable to enzymes or other macromolecules and optionally are impermeable to water. Macromolecules are defined herein as molecules with a molecular weight greater than 10,000 Daltons.

In one aspect, the present invention is particularly suited for the administration of musculotropic or neurochemicals to smooth muscle tissues. Tissues and organs that can benefit from the present invention include, but are not limited to, stomach, intestines, heart, uterine, bladder, ureter, urethra, sphincter muscles, and the esophagus.

The present invention is designed to reduce or eliminate incontinence frequency with pure detrusor instability or with combination of stress urinary incontinence and detrusor instability without any upper motor neuron lesion. The device can effectively deliver therapeutic agents for at least six months and is reimplantable. The implant can be performed in the doctor's office or on an outpatient basis.

The present invention provides a device for localized long-term administration of a therapeutic agent to a particular tissue or organ.

The device is useful for the long-term delivery of a musculotropic or neurochemical to smooth muscle tissue.

The device is useful for incontinent patients with bladder instability etiology.

These features and advantages will become apparent after a review of the following detailed description of the disclosed embodiments and the appended claims.

### Brief Description of the Figures

Fig. 1 is a side view of an apparatus useful for the long-term delivery of a therapeutic agent to a tissue or organ.
Fig. 2 is a cross-sectional view of the apparatus shown in Fig. 1.

### Detailed Description

The present invention consists of an apparatus for the localized, long-term treatment of a tissue or organ. The present invention is especially suited for the long-term treatment of smooth muscle tissues and organs.

The present invention, in one embodiment, is an apparatus comprising a container containing a therapeutic agent that has been physically trapped, or covalently or ionically immobilized in a biodegradable matrix. The therapeutic agents can be physically trapped in the matrix by mixing, ionic bonding on the matrix and/or covalently bonded via irradiation or through the use of cross-linkers including, but not limited to, glutaraldehyde, polyethyleneglycol epoxide or ethylene oxide. In a preferred embodiment, the biodegradable matrix is in the form of a cylinder. The sides of the cylinder are preferably an impermeable coating. One or both ends of the cylinder can be open to the tissue, or the cylinder ends may be covered with a water-permeable membrane.

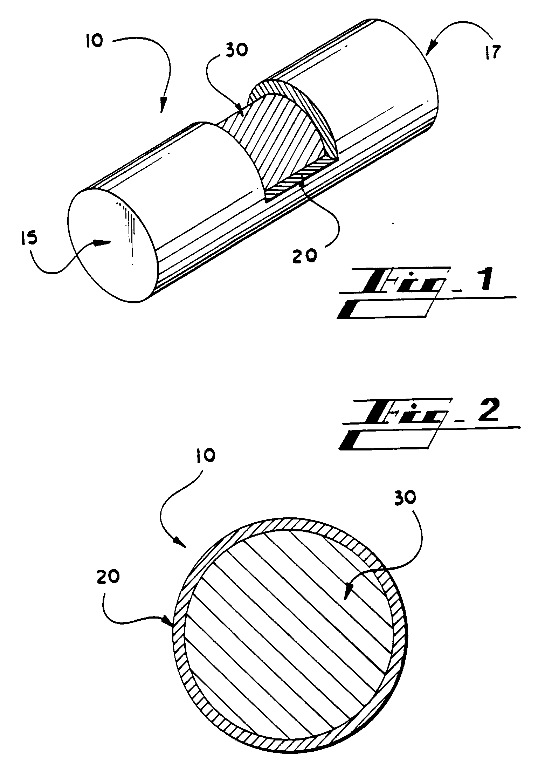

As shown in Fig. 1, one embodiment of the present invention is a cylinder **10** comprising a wall **20** that is preferably non-permeable to macromolecules such as proteins and cells. The cylinder wall **20** can be made of medical-grade silicone, stainless steel, titanium, or plastics. The cylinder **10** is filled with a biodegradable matrix **30** with a therapeutic admixed therein. The ends **15**, **17** of the cylinder **10** are preferably open to the tissue. Alternatively, the ends **15**, **17** of the cylinder can be covered with a membrane that is permeable to water, macromolecules and optionally to cells. In another embodiment of the present invention, one end **15** of the cylinder **10** can be sealed with a material that is impermeable to enzymes and cells and the other end of the cylinder **10** can be open to tissue. Fig. 2 is a cross-sectional view of cylinder **10** showing the cylinder walls **20** and the biodegradable matrix **30** therein.

In a preferred embodiment of the present invention, the device is preferably between 0.1 cm and 3.0 cm in length and preferably between 0.1 cm and 3.0 cm in diameter. It is to be understood that while a cylinder is the preferred shape of the device that is contemplated as the present invention, the device can be any other shape including, but not limited to, a disc, or a ring.

The biodegradable matrix that is placed inside the cylinder is a matrix chosen from biocompatible materials such as liposomes, polylactides (polylactic acid), polyglycolide (polymer of glycolic acid), poly(lactide co-glycolide) (copolymers of lactic acid and glycolic acid), polyanhydrides, polypeptides, hyaluronic acid, collagen, chondroitin sulfate, carboxylic acids, fatty acids, phospholipids, polysaccharides, nucleic acids, amino acids such as phenylalanine, tyrosine, isoleucine, polynucleotides, polyethylene glycol polyvinyl propylene, polyvinylpyrrolidone and beeswax.

The therapeutic agents can be any compound that is biologically active and requires long term administration to a tissue or organ for maximum efficacy. Therapeutic agents that can be used in accordance with the present invention include, but are not limited to, antimuscarinic agents, anticholinergic agents, antispasmodic agents, calcium antagonist agents, potassium channel openers, musculotropic relaxants, antineoplastic agents, polysynaptic inhibitors, and beta-adrenergic stimulators. Examples of anticholinergic agents are propantheline bromide, imipramine, mepenzolate bromide, isopropamide iodide, clidinium bromide, anisotropine methyl bromide, scopolamine hydrochloride, and their derivatives. Examples of antimuscarinic agents include, but are not limited to, hyoscyamine sulfate, atropine, methantheline bromide, emepronium bromide, anisotropine methyl bromide, and their derivatives. Examples of polysynaptic inhibitors are baclofen and its derivatives. Examples of β-adrenergic stimulators are terbutaline and its derivatives. Examples of calcium antagonists are terodiline and its derivatives. Examples of musculotropic relaxants include, but are not limited to, dicyclomine hydrochloride, flavoxate hydrochloride, papaverine hydrochloride, oxybutynin chloride, and their derivatives. Examples of an antineoplastic agents include, but are not limited to, carmustine levamisole hydrochloride, flutamide, (w-methyl-N-[4-nitro-3-(trifluoromethyl) phenyl]), adriamycin, doxorubicin hydrochloride, idamycin, fluorouracil, cytoxan, mutamycin, mustargen and leucovorin calcium.

It is important that the kinetics of the therapeutic agent release be zero order kinetics or near zero order kinetics. The rate of release of the therapeutic agent will vary depending upon the target tissue or organ and on the therapeutic agent or agents being delivered. The rate of release of the therapeutic agent can be controlled by the choice of active ingredients with different solubilities and biodegradable matrix, how the therapeutic agent is physically trapped or chemically immobilized in the biodegradable matrix, by varying the area of the biodegradable matrix exposed to the tissue by adjusting the area of the opening in the container, and/or adjusting the permeability of the walls and/or opening to the therapeutic agent.

Musculotropic relaxants are direct acting smooth muscle depressants and act directly on the smooth muscle at sites distal to the cholinergic receptors. β-adrenergic stimulators stimulate the relaxation mediating beta receptors in smooth muscle. Calcium antagonists relax many types of smooth muscle. The calcium antagonist would decrease or inhibit the influx of calcium ion into smooth muscles and/or activate the calcium pump to pump calcium ions out of smooth muscle into extracellular fluids. Because all types of smooth muscle have poorly developed sarcoplasmic reticuli for storing calcium ions, the calcium source for smooth muscle relaxation is always the extracellular fluid. Antimuscarinic inhibitors prevent the effects of parasympathetic stimulation at the neuroeffector junction. These antimuscarinic agents result in parasympathetic stimulation at the neuroeffector junction. Polysynaptic inhibitors relax smooth muscle by acting on nerve cells in the neural conduction pathways formed by a chain of many synaptically connected nerve cells.

Fiber (0.2 mm in diameter) made of polyglycolide/oxybutynin chloride and polylactide/oxybutynin chloride are extruded into and out of silicone room temperature vulcanization (RTV) for coating. The coated fibers are cut into 0.5 mm segments. The segments of polyglycolide/oxybutynin chloride fiber and segments of polylactide/oxybutynin chloride fiber are mixed in USP grade glycerol. Then the suspended material is injected into the bladder submucosa endoscopically using a 16 gauge transurethral catheter needle.

The invention is further illustrated by the following examples, which are not to be construed in any way as imposing limitations upon the scope thereof.

### Example I

Polylactide and oxybutynin chloride powder (5:1 by weight) are blended by mechanical stirring and extruded at approximately 140°C to form a long cable (3-mm in diameter). The cable is cut into 1 inch pellets. One end of these pellets is blocked with a biocompatible silicone (RTV) adhesive. With the blocked end facing downwards, the pellets are placed on a turntable inside a vacuum chamber. After desirable vacuum is reached (approximately 10⁻¹⁰ mBar), the DC current is turned on and the voltage is increased until sparks occur at the 24K gold wire electrode. The gold wire electrode must be positioned above the top of the pellets and at an angle to them. The gold is spattered on the surface of the rotating rods until a minimum of 0.01µm (100Å) thick gold coating is achieved. The adhesive top is then removed by either peeling or mechanical abrasion.

### Example II

Polyglycolide and hyoscyamine sulfate powder (20:1 by weight) are blended by stirring and extruded at approximately 160°C to form a 5-inch rod (1 mm in diameter). The rod is rotated around its axis while it is coated with pure platinum by vacuum deposit to reach a 10 µm thickness. Then the rod is cut into half-inch segments. An 18-gauge, 8,9 cm (3 1/2 inch) needle with a stylet is used to deliver the pellet directly into the bladder neck tissue of a female patient. The needle with a stylet in place is inserted perineally and parallel to the inserted cystoscope. With the aid of the scope, the tip of the needle is located while it is advanced toward the bladder neck. When the tip is approximately one inch from the bladder neck, the stylet is removed while holding the needle immobile. Approximately 0.3 cm³ of USP grade glycerol is injected into the needle lumen. The 12,7 mm (1/2inch) rod is then inserted into the lumen of the needle, then the rod is pushed into the bladder neck tissue using the stylet. The glycerol functions as a lubricant and protective layer during the insertion of the rod into the bladder neck tissue.

### Example III

Polyanhydride, a copolymer of bis (p-carboxyphenoxy) propane and sebacic acid at a ratio of 20:80 is mixed with Terodiline ((N-(1,1 -dimethylethyl)-α-methyl-γ-phenylbenzenepropanamine). The mixture is then molded into 10 mm by 3 mm pellets. The pellets are coated with gold as described in Example I. One end is not blocked. A trocar-sleeve system is used to deliver the pellet to the periurethral tissue. The Terodiline is delivered from the coated pellet at a zero order kinetic rate of ≥1.7 µg/hour.

### Example IV

Polyanhydride copolymer is mixed with the antineoplastic agent carmustine (N,N-bis(2-chloroethyl)-N-nitroso-urea) and atropine. A pellet is formed with the dimensions of 10 mm (L) by 4 mm (D). The pellets are coated with gold on the sides and on one end. One end is left open. After a cancer patient's prostatic tumor is removed, one pellet is left at the surgical site prior to suture. When the carmustine and atropine are exhausted from the pellet, a second pellet can be delivered, if necessary, through a working channel of an endoscope.

### Example V

A stainless steel cylindrical container with inner and outer dimensions of 1.5 cm (L) x 1.0 cm (inside measurements) x 1.7 cm (L) x 1.4 cm (outside measurements) respectively is packed with propantheline and polylactic acid (1:50) and compressed under pressure. The open end is sealed with a nitrocellulose membrane. This cylindrical device is then implanted either surgically or through an endoscope into the stomach wall of a cow suffering from chronic emetics.

### Example VI

Fibers (0.2 mm in diameter) made of polyglycolide/propantheline(80:20) and polylactide/methscopolamine (50:50) are extruded into silicone (RTV) for coating. Then the two coated fibers are cut into 1.0 mm segments. The segments of polyglycolide/propantheline fiber and segments of polylactide/ methscopolamine fiber are mixed in USP grade glycerol. The suspended material is then injected endoscopically into the stomach wall of cattle with emetics using a 14-gauge catheter needle.

## Claims

1. An implant device for the long term delivery of a therapeutic agent to a tissue or organ comprising
(i) a container (10) having a wall (20) for implantation within the tissue or organ and
ii) a therapeutic agent immobilized in a matrix (30) contained within the container (10) such that the therapeutic agent is released into the tissue or organ at a rate of at least approximately 0.1 µg/day over a period of at least approximately three months; **characterized in that**
the matrix (30) is biodegradable and selected from liposomes, polylactides, polyanhydrides, polyglycolide, poly(lactide co-glycolide), polypeptides, hyaluronic acid, collagen, chondroitin sulfate, carboxylic acids, fatty acids, phospholipids, polysaccharides, nucleic acids, phenylalanine, tyrosine, isoleucine, polynucleotides, polyethylene glycol, polyvinylpropylene, polyvinylpyrrolidone and beeswax.

2. The implant device of claim 1, wherein the container wall (20) is of a material which is impermeable to macromolecules and at least a portion of said container (10) being permeable to the therapeutic agent.

3. The implant device of claim 2, wherein the rate of release of the therapeutic agent is controlled by controlling, the permeability of the permeable portion of said container (10).

4. The implant device of claim 2, wherein the rate of release of the therapeutic agent is controlled by controlling the dimensions of the permeable portion of said container (10).

5. The implant device of claim 1, wherein the rate of release of the therapeutic agent is controlled by controlling the rate of biodegradation of the biodegradable matrix (30).

6. The implant device of claim 1, wherein the therapeutic agent is selected from the group consisting of antimuscarinic agents, anticholinergic agents, antispasmodic agents, calcium antagonist agents, potassium channel openers, musculotropic relaxants, polysynaptic inhibitors, beta-adrenergic stimulators and antitumor agents.

7. The implant device of claim 1, wherein the therapeutic agent is selected from the group consisting of propantheline bromide, imipramine, hyoscyamine sulfate, oxybutynin chloride, carmustine, dicyclomine and terodiline.

8. The implant device of claim 2, wherein the container wall (20) is selected from silicone, stainless steel, gold, titanium, or medical/surgical grade plastics.

9. The implant device of claim 8, wherein the plastics are selected from polyethylene, polypropylene and parylene.

10. The implant device of claim 1, wherein the container (10) is a cylinder.

11. The implant device of claim 10, wherein the cylinder has a diameter between 0.1 and 3.0 cm and a length between 0.1 cm and 3 cm.

## Patentansprüche

1. Implantatvorrichtung für die Langzeitfreisetzung eines therapeutischen Mittels an ein Gewebe oder ein Organ, enthaltend:
(i) einen Behälter (10) mit einer Wand (20) zur Implantation innerhalb des Gewebes oder Organs und
(ii) ein therapeutisches Mittel, das in einer Matrix (30), die innerhalb des Behälters (10) enthalten ist, immobilisiert ist, so daß das therapeutische Mittel in das Gewebe oder das Organ mit einer Geschwindigkeit von wenigstens ungefähr 0,1 µg/Tag über einen Zeitraum von wenigstens ungefähr 3 Monaten freigesetzt wird,
dadurch gekennzeichnet, daß die Matrix (30) bioabbaubar ist und ausgewählt ist aus Liposomen, Polylactiden, Polyanhydriden, Polyglykolid, Poly(lactidcoglykolid), Polypeptiden, Hyaluronsäure, Collagen, Chondroitinsulfat, Carbonsäuren, Fettsäuren, Phospholipiden, Polysacchariden, Nucleinsäuren, Phenylalanin, Tyrosin, Isoleucin, Polynucleotiden, Polyethylenglykol, Polyvinylpropylen, Polyvinylpyrrolidon und Bienenwachs.

2. Implantatvorrichtung nach Anspruch 1, wobei die Behälterwand (20) ein Material ist, das für Makromoleküle undurchlässig ist, und wenigstens ein Teil dieses Behälters (10) durchlässig für das therapeutische Mittel ist.

3. Implantatvorrichtung nach Anspruch 2, wobei die Freisetzungsgeschwindigkeit für das therapeutische Mittel durch Regelung der Permeabilität des permeablen Anteils dieses Behälters (10) gesteuert wird.

4. Implantatvorrichtung nach Anspruch 2, wobei die Freisetzungsgeschwindigkeit für das therapeutische Mittel durch Regelung der Dimensionen des permeablen Anteils dieses Behälters (10) gesteuert wird.

5. Implantatvorrichtung nach Anspruch 1, wobei die Freisetzungsgeschwindigkeit für das therapeutische Mittel durch Regelung der Geschwindigkeit des Bioabbaus der bioabbaubaren Matrix (30) gesteuert wird.

6. Implantatvorrichtung nach Anspruch 1, wobei das therapeutische Mittel ausgewählt ist aus der Gruppe bestehend aus antimuscarinen Mitteln, anticholinergischen Mitteln, krampflösenden Mitteln, Calciumantagonistmitteln, Kaliumkanalöffnern, musculotropen Entspannungsmitteln, polysynaptischen Inhibitoren, beta-adrenergischen Stimulatoren und Antitumormitteln.

7. Implantatvorrichtung nach Anspruch 1, wobei das therapeutische Mittel ausgewählt ist aus der Gruppe bestehend aus Propanthelinbromid, Imipramin, Hyoscyaminsulfat, Oxybutininchlorid, Carmustin, Dicyclomin und Terodilin.

8. Implantatvorrichtung nach Anspruch 2, wobei die Behälterwand (20) ausgewählt ist aus Silicon, Edelstahl, Gold, Titan oder Kunststoffen für medizinische oder chirurgische Verwendung.

9. Implantatvorrichtung nach Anspruch 8, wobei die Kunststoffe ausgewählt sind aus Polyethylen, Polypropylen und Parylen.

10. Implantatvorrichtung nach Anspruch 1, wobei der Behälter (10) ein Zylinder ist.

11. Implantatvorrichtung nach Anspruch 10, wobei der Zylinder einen Durchmesser zwischen 0,1 und 3,0 cm und eine Länge zwischen 0,1 cm und 3 cm hat.

## Revendications

1. Implant pour la délivrance à long terme d'un agent thérapeutique à un tissu ou un organe, comprenant
(i) un réservoir (10) ayant une paroi (20) pour l'implantation dans le tissu ou l'organe et
(ii) un agent thérapeutique immobilisé dans une matrice (30) contenue dans le réservoir (10) de façon que l'agent thérapeutique soit libéré dans le tissu ou l'organe à une vitesse d'au moins environ 0,1 µg/jour pendant une période d'au moins environ 3 mois,
caractérisé en ce que la matrice (30) est biodégradable et choisie parmi des liposomes, des polymères d'acide lactique, des polyanhydrides, des polymères d'acide glycolique, des copolymères d'acide lactique et d'acide glycolique, des polypeptides, l'acide hyaluronique, le collagène, le sulfate de chondroïtine, des acides carboxyliques, des acides gras, des phospholipides, des polysaccharides, des acides nucléiques, la phénylalanine, la tyrosine, l'isoleucine, des polynucléotides, le polyéthylèneglycol, le polyvinylpropylène, la polyvinylpyrrolidone et la cire d'abeilles.

2. Implant selon la revendication 1, dans lequel la paroi (20) du réservoir est constituée d'un matériau qui est imperméable aux macromolécules et au moins une portion dudit réservoir (10) est perméable à l'agent thérapeutique.

3. Implant selon la revendication 2, dans lequel la vitesse de libération de l'agent thérapeutique est contrôlée par le réglage de la perméabilité de la portion perméable dudit réservoir (10).

4. Implant selon la revendication 2, dans lequel la vitesse de libération de l'agent thérapeutique est contrôlée par le réglage des dimensions de la portion perméable dudit réservoir (10).

5. Implant selon la revendication 1, dans lequel la vitesse de libération de l'agent thérapeutique est contrôlée par le réglage de la vitesse de biodégradation de la matrice biodégradable (30).

6. Implant selon la revendication 1, dans lequel l'agent thérapeutique est choisi dans le groupe constitué par des agents antimuscariniques, des agents anticholinergiques, des agents antispasmodiques, des agents antagonistes du calcium, des agents ouvrant les vannes à potassium, des relaxants myotropiques, des inhibiteurs polysynaptiques, des stimulants β-adrénergiques et des agents antitumoraux.

7. Implant selon la revendication 1, dans lequel l'agent thérapeutique est choisi dans le groupe constitué par le bromure de propanthéline, l'imipramine, le sulfate d'hyoscyamine, le chlorure d'oxybutynine, la carmustine, la dicyclomine et la térodiline.

8. Implant selon la revendication 2, dans lequel la paroi (20) du réservoir est choisie parmi une silicone, l'acier inoxydable, l'or, le titane ou des matières plastiques de qualité médicale/chirurgicale.

9. Implant selon la revendication 8, dans lequel les matières plastiques sont choisies parmi le polyéthylène, le polypropylène et le parylène.

10. Implant selon la revendication 1, dans lequel le réservoir (10) est un cylindre.

11. Implant selon la revendication 10, dans lequel le cylindre a un diamètre compris entre 0,1 et 3,0 cm et une longueur comprise entre 0,1 cm et 3 cm.
